(19) **Europäisches Patentamt**

**European Patent Office**

**Office européen des brevets**

(11) **EP 1 205 540 A1**

(12) **EUROPÄISCHE PATENTANMELDUNG**

(43) Veröffentlichungstag:
**15.05.2002 Patentblatt 2002/20**

(51) Int Cl.$^7$: **C12M 1/34**

(21) Anmeldenummer: **00124662.8**

(22) Anmeldetag: **10.11.2000**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder: **Evotec OAI AG**
**22525 Hamburg (DE)**

(72) Erfinder:
- **Gradl, Gabriele Dr.**
**10557 Berlin (DE)**

- **Schnelle, Thomas Dr.**
**10843 Berlin (DE)**
- **Müller, Torsten Dr.**
**12439 Berlin (DE)**
- **Reichle, Christoph**
**13353 Berlin (DE)**

(74) Vertreter: **Hertz, Oliver, Dr.**
**v. Bezold & Partner,**
**Patentanwälte**
**Akademiestrasse 7**
**80799 München (DE)**

(54) **Verfahren zur Vitalitätsmessung an Zellen**

(57) Es wird ein Verfahren zur zerstörungsfreien Vitalitätsmessung an biologischen Zellen, insbesondere zur Bestimmung von Apoptose beschrieben bei dem mindestens eine Zelle hochfrequenten alternierenden, insbesondere rotierenden, elektrischen Feldern und/oder Impedanzprüffeldern ausgesetzt wird und an der Zelle für mindestens einen Frequenzbereich oder einzelne Frequenzen mindestens eine Rotationsmessung, eine Dielektrophoresemessung und/oder eine Impedanzmessung erfolgen, aus denen mindestens ein Messparameter ermittelt wird, der für den Vitalitätszustand der Zelle charakteristisch ist.

EP 1 205 540 A1

Printed by Jouve, 75001 PARIS (FR)

**Beschreibung**

Die Erfindung betrifft ein Verfahren zur zerstörungsfreien Vitalitätsmessung an biologischen Zellen, insbesondere zur zerstörungsfreien Bestimmung von Apoptose an Zellen. Das erfindungsgemäße Verfahren erlaubt es ferner, apoptotische Zellen von vitalen und nekrotisch geschädigten zu unterscheiden. Weiterhin betrifft die vorliegende Erfindung ein Verfahren zur Identifizierung von Apoptose beeinflussenden Substanzen.

Zelltod tritt in der Natur in zwei unterschiedlichen Erscheinungsformen auf, der Nekrose und der Apoptose. Die Nekrose ist der Zelltod durch eine unspezifische physikalische oder chemische Schädigung. Durch Verletzung der Barrierefunktion der Plasmamembran wird ihre Semipermeabilität für Ionen (besonders $Ca^{2+}$, $Na^+$, $K^+$) gestört. Dadurch entsteht ein massiver Ionen- und Lösungseinstrom in die Zelle, in folge dessen sich die Zelle aufbläht und schließlich platzt. Die Kompartimentierung der Zelle wird somit aufgehoben und der Zellinhalt ausgeschüttet. Dieser Vorgang löst nun im Organismus Entzündungsreaktionen aus. In der Regel erfolgen nekrotische Schädigungen innerhalb von Sekunden bis Minuten.

Im Gegensatz dazu handelt es sich bei der Apoptose um einen programmierten Zelltod. Apoptose ist ein organisierter Prozeß, der in der Entwicklung und Erhaltung eines Organismus benötigt wird, um unerwünschte oder schadhafte Zellen zu eliminieren, ohne den Gesamtorganismus zu schädigen. Sie wird durch Rezeptoren kontrolliert (Glucocorticoid-R, TNFR, Fas, NGFR) und ist in der Regel von einer de-novo Proteinsynthese abhängig. Es werden zelluläre Proteasen (ICE, Caspasen) und Endonukleasen aktiviert. Da nahezu alle Zellen eines Organismus Apoptose durchlaufen können, muß der Auslöser des Apoptoseprozesses unter permanenter und sorgfältiger physiologischer Kontrolle stehen. Rein phänomenologisch kann die Apoptose in sechs aufeinanderfolgende Stadien unterteilt werden: 1. Schrumpfung, 2. Zeiosis (Plasmamembranprotuberanzen), 3. Chromatinkollaps, 4. Kollabieren des Zellkerns, 5. Aufbrechen des Zellkerns in kleinere Einheiten und Fragmentierung der zellulären DNA, 6. Abschnüren von apoptotischen Vesikeln (Thompson, C. B., 1995, Apoptosis in the pathogenesis and treatment of disease, Science 267: 1456-1462). Während des gesamten Prozesses bleibt die Barrierefunktion der Plasmamembran erhalten. Die Auslösung der Apoptose erfolgt sowohl durch interne Signale des Organismus als auch durch äußere Einflüsse, wie z. B. Strahlung, chemische Substanzen oder Wirkstoffe. Die Apoptose ist ein Vorgang, bei dem erste Anzeichen sich manchmal erst nach Stunden bemerkbar machen. Apoptotische Zellen werden im Gegensatz zu nekrotischen Zellen durch benachbarte Zellen oder Makrophagen erkannt und entfernt (Orrenius, S., 1995, Apoptosis: molecular mechanisms and implications for human disease, J. Internal Medicine 237:529-536). Eine Entzündungsreaktion tritt hier nicht auf.

Neuere Studien belegen, daß Störungen in der Regulation der Apoptose einer Vielzahl von Erkrankungen zu Grunde liegt. Krankheiten die mit einer Blockierung der Apoptose in Verbindung gebracht werden, sind insbesondere Krebserkrankungen, wie z. B. Carcinome mit p53 Mutationen, hormon-abhängige Tumore; Autoimmun-Krankheiten, wie z. B. rheumatische Arthritis, Diabetis mellitus; und virale Infektionen, wie z. B. Infektionen durch Herpesviren, Poxviren, Adenoviren. Krankheiten die vermutlich durch eine erhöhte Apoptoserate verursacht werden, sind insbesondere AIDS; neurodegenerative Erkrankungen, wie z. B. Alzheimer, Parkinson; durch Toxin, wie z. B. große Alkoholmengen verursachte Lebererkrankungen (Thompson, C. B., 1995, Apoptosis in the pathogenesis and treatment of disease, Science 267:1456-1462).

Zur Bestimmung der Apoptose wurden daher bereits eine Vielzahl von Verfahren auf der Basis apoptosespezifischer Parameter entwickelt. Beispielsweise basieren diese Methoden auf der Bestimmung des Phosphatidylserin-Anteils auf der extrazellulären Zelloberfläche durch die Bindung von Annexin V, die Bestimmung von hypodiploiden Zellkernen, der Bestimmung der cytosolischen Cytochrom C- oder Caspase-Konzentration oder der Bestimmung der Caspase-Aktivierung.

Die Detektion erfolgt durch mikroskopische, fluoreszenzbasierende und biochemische Verfahren. Die Elektronenmikroskopie und die Phasenkontrast-Mikroskopie erfassen beispielsweise die morphologischen Erscheinungen beim Ablauf der Apoptose, wie Schrumpfung, Membranprotuberanzen und das Auftreten von Mikronuklei (S. Verhaegen 1998, Microscopical Study of Cell Death Via Apoptosis, European Microscopy and Analysis). Allerdings erfordert die Quantifizierung der Apoptose in einer Probe über Mikroskopie ein hohes Maß an Erfahrung des Experimentators im Urteilsvermögen und ein manuelles Auszählen der Zellen nach optischer Beurteilung oder ein geeignetes Bildverarbeitungsprogramm.

Ein weiteres bekanntes Verfahren zur Erfassung von Apoptose in einer Probe suspendierter Zellen erfordert die Verwendung eines Durchflußzytometers. Hier werden Fluoreszenzfarbstoffe, die DNA anfärben, verwendet (K. H. Elstein and R. M. Zucker 1994, Comparison of cellular and Nuclear Flow Cytometric Techniques for Discriminating Apoptotic Subpopulations, Experimental Cell Research 211, 322-331) oder es wird ein biochemisches Verfahren eingesetzt, bei dem der Prozeß der DNA-Fragmentierung durch den Einbau fluoreszenzmarkierter Nukleotide sichtbar gemacht wird (z. B. TUNEL-Methode, R. S. Douglas, A. D. Tarshis, C. H. Pletcher, P. C. Nowell und J. S. Moore, 1995, A simplified method for the coordinate examination of apoptosis and surface phenotype of murine lymphocytes, Journal of Immunological Methods 188, 219-228). Ebenso werden für Zelloberflächenmoleküle spezifische fluoreszenzmarkierte Sonden eingesetzt. Ein Beispiel für eine solche Sonde ist fluoreszenzmarkiertes Annexin-V (G. Koopman, C.P.

M. Reutlingsperger, G.A.M. Kuijten, R.M.J. Keehnen, S.T. Pals und M.H.J. van Oers, 1994, Annexin V for Flow Cytometric Detection of Phosphatidylserine Expression on B Cells Undergoing Apoptosis, Blood 84,5, 1415-1420), welches an Phosphatidylserin auf der Zelloberfläche bindet und so eine Umstrukturierung der Plasmamembran im Frühstadium der Apoptose sichtbar macht. Allerdings erfordern alle durchflusszytometrischen-Methoden zur Messung der Apoptose eine Kalibrierung der Methode und des Systems. Zudem ist eine hohe Anzahl von Zellen (ca. $10^6$) für eine Analyse notwendig.

Weitere Methoden zum Nachweis der Apoptose erfordern die Zerstörung aller Zellen einer Probe und den gelelektrophoretischen oder biochemischen Nachweis der DNA-Fragmentierung (M. Leist, F. Gantner, I. Bohlinger, P. G. Germann, G. Tiegs und A. Wendel, 1994, Murine Hepatocyte Apoptosis Induced In Vitro and In Vivo by TNF-a Requires Transcriptional Arrest,The Journal of Immunology 153, 1778-1788). Es handelt sich bei diesen biochemischen Methoden um mehrstufige Prozesse unter Verwendung von mehreren Reagenzien. Dies führt zu einer sehr langen Analysendauer von bis zu 6 Stunden. Zudem werden mehrere hundert Zellen für eine Analyse benötigt.

Durch die Relevanz einer gestörten Regulation der Apoptose im Zusammenhang mit einer Vielzahl von Erkrankungen ist die Untersuchung der Apoptose auch ein zentraler Bestandteil bei der Suche nach neuen pharmazeutischen Wirkstoffen, bei der Beurteilung ihrer Wirksamkeit gegenüber Erkrankungen sowie in der Umweltanalytik. Generell muss eine Apoptose-beeinflussende Verbindung nicht nur dazu in der Lage sein, Apoptose zu modulieren, sondern auch die intakte Zellmembran zu durchdringen.

Darüber hinaus wird angenommen, daß eine Vielzahl von zellulären Rezeptoren, Proteinen, Zellbestandteilen und Kofaktoren den Prozeß der Apoptose in lebenden Zellen beeinflusst.

Es wurde daher bereits eine Vielzahl von Screeningverfahren zur Identifizierung Apoptose-beeinflussender Substanzen, die auf der Bestimmung Apoptose-spezifischer Parameter in der Zelle oder in zellfreien Systemen beruhen, entwickelt.

So beschreibt beispielsweise WO 98/02579 einen Screening Assay zur Identifizierung von Apoptose-regulierenden Substanzen in einem zellfreien System. Zur Bestimmung von Apoptose werden hier verschiedene Apoptose-spezifische Parameter, wie beispielweise Cytochrome C oder CPP32 Protease-Aktivität bestimmt. Zum Screenen wurde ein bei 100000 x g gewonnener Überstand vom Cytosol nicht-apoptotischer Zellen eingesetzt. Die Bestimmung von Apoptose-beeinflussenden Substanzen wurde durch Zugabe von Testverbindungen, die einen negativen, positiven oder keinen Einfluß auf die Apoptose haben können, zu diesem Überstand, durchgeführt. Die so erzielten Ergebnisse werden mit denen einer Referenz verglichen.

WO 98/55615 offenbart einen Screening Assay zur Bestimmung von therapeutisch wirksamen Substanzen, die Apoptose-beeinflussen. Es werden zellfreie Assays beschrieben, die so ausgelegt sind, Verbindungen zu untersuchen, die mit Cytochrom C um die Bindung an Apaf-1 konkurrieren. Darüber hinaus werden auch Assays zur Bestimmung Apoptose-beeinflussenden Substanzen beschrieben, die auf der Untersuchung der proteolytischen Spaltung von Caspase-3 Vorläufern in Gegenwart und Abwesenheit von potentiellen Apoptose beeinflussenden Substanzen bestimmt.

In WO 99/18856 wird ein Verfahren zur Auffindung von Substanzen zur Anregung oder Inhibierung von Enzymen der Apoptose-Kaskade, insbesondere Caspasen, beschrieben. Das Verfahren wird mit ganzen Zellen oder Geweben durchgeführt. Hierzu wird ein Teil der Zellen mit den Testverbindungen und fluoreszenzmarkierten Reportermolekülen behandelt, wohingegen ein anderer Teil zur Kontrolle nur mit den Reportern behandelt wird. Eine Änderung der Fluoreszenz im Gegensatz zur Kontrollprobe zeigt an, daß eine zu untersuchende Substanz Enzyme der Kaskade beeinflußt.

Insgesamt weisen jedoch alle bisher bekannten Verfahren zur Bestimmung von Apoptose oder Apoptose modulierenden Substanzen eine Vielzahl von Nachteilen auf. So sind sie oft sehr zeitaufwendig aufgrund von aufwendigen experimentellen Schritten und benötigen zudem eine große Menge an z.B. Antikörpern, Farbstoffen und weiteren Reagenzien, die in die Zelle eindringen können. Darüberhinaus basieren die meisten Verfahren auf einer abschließenden Lyse der Zellen, was letztendlich zu einer Zerstörung der Zellen führt. Andere untersuchte Parameter, wie z.B. Annexin V sind nicht Apoptose-spezifisch, wieder andere Verfahren benötigen große Mengen an Zellen. Darüber hinaus sind viele Verfahren nur geeignet, späte Stadien der Apoptose zu untersuchen, die häufig in vivo nicht auftreten, da die apoptotischen Zellen in diesem Stadium bereits durch Phagozytose von benachbarten Zellen oder Makrophagen aufgenommen wurden.

Häufig eingesetzte zellfreie Assays wiederum, wie beispielweise zur Bestimmung von Cytochrom C und anderen Apoptosespezifischen Markern sind nicht dazu geeignet, die Fähigkeit von Apoptose-beeinflussenden Parametern, die intakte Zellwand zu passieren, zu bestimmen.

Darüber hinaus wird angenommen, daß verschiedene Zelltypen unterschiedliche Rezeptoren und Kofaktoren zur Apoptose Modulation besitzen. Es ist aus diesem Grund nicht möglich, mit zellfreien-Assays Zelltyp-spezifische oder organspezifische Apoptose-Modulatoren zu finden.

Durch die Vernachlässigung der Bedeutung der zellulären Rezeptoren und anderer Kofaktoren durch die Mehrheit der bekannten Assays ist die Gefahr der Identifizierung von falsch-positiven oder falsch-negativen Apoptose-beeinflussenden Substanzen hoch, da diese Substanzen in lebenden Zellen keine oder nicht die erwartete Wirkung zeigen. Zudem werden Apotosebeeinflussende Substanzen, die Apoptose indirekt über einen dieser Rezeptoren oder Kofaktoren modulieren, nicht erfaßt.

Unter Beachtung der beschriebenen Nachteile der bekannten Verfahren ist es wünschenswert, ein zur eindeutigen Identifizierung von Apoptose und der Bestimmung von Apoptosemodulierenden Substanzen geeignetes Verfahren zur Verfügung zu haben. Dieses Verfahren sollte dazu in der Lage sein, Apoptose in einem frühen Stadium zu erkennen, einfach und mit ganzen Zellen durchführbar sein.

Dielektrophoretische Techniken, wie z. B. die dielektrische Einzelspektroskopie (R. Pethig und G. H. Markx, 1997, Applications of dielectrophoresis in biotechnology, Trends in Biotechnology 15, 426-432) und die Elektrorotation (T. Schnelle, T. Müller und G. Fuhr,1999, Dielectric single particle spectroscopy for measurement of dispersion, Medical & Biological Engineering & Computing 37, 264-271) sind an sich bereits bekannt.

Es sind auch bereits Vorrichtungen zur Messung der Elektrorotation unter Zuhilfenahme einer optischen Pinzette (T. Schnelle, T. Müller, C. Reichle und G. Fuhr 2000, Combined dielectrophoretic field cages and laser tweezers for electrorotation, Applied Physics B, Lasers and Optics, Springer-Verlag) und einer automatischen Signalaufnahme (C. Reichle, T. Müller, T. Schnelle und G. Fuhr 1999, Electro-rotation in octopole micro cages, Journal of Physics D: Applied Physics 32, 2128-2135; De Gasperis, Wang, Yang, Becker und Gascoyne 1998, Meas. Sci. Technol. 9, 518-529) bekannt.

Die Aufgabe der vorliegenden Erfindung ist es, ein verbessertes Verfahren zur zerstörungsfreien Vitalitätsmessung, insbesondere zur Erfassung von Apoptose, an biologischen Zellen bereitzustellen. Die Erfindung soll insbesondere die Bereitstellung eines verbesserten Verfahrens zur Identifizierung von Apoptose-beeinflussenden Substanzen ermöglichen.

Diese Aufgabe wird durch Verfahren mit den Merkmalen gemäß Patentanspruch 1 gelöst. Vorteilhafte Ausführungsformen und Anwendungen der Erfindung ergeben sich aus den abhängigen Ansprüchen.

Die Grundidee der Erfindung besteht in der Bereitstellung eines Verfahrens zur zerstörungsfreien Vitalitätsmessung an Zellen durch Einbringen mindestens einer zu untersuchenden Zelle in ein Mikrosystem, bei dem die mindestens eine Zelle hochfrequenten rotierenden elektrischen Feldern oder elektrischen Wechselfeldern, insbesondere Impedanzprüffeldern, ausgesetzt ist.

Die Erfindung beinhaltet in bevorzugten Ausführungsformen Verfahren zur Feststellung der Apoptose in suspendierten Zellen durch dielektrophoretische Techniken.

Eine Unterscheidung insbesondere von vitalen, nekrotischen und apoptotischen Zellen ist mit den erläuterten herkömmlichen Verfahren nicht möglich.

Darüber hinaus weist das erfindungsgemäße Verfahren viele Vorteile gegenüber den bereits bekannten Verfahren auf. So entfällt beispielsweise die Notwendigkeit von spezifischen Nachweisreagenzien sowie der dadurch bedingte Zeitaufwand bei der Probenaufbereitung durch oft mehrstufige Prozesse, wie auch aufwendige Bildanalysen. Das erfindungsgemäße Verfahren besitzt zudem Einzelzell-Sensitivität, Spezifität für den apoptotischen Prozeß, ermöglicht die Unterscheidung von nekrotischen und apoptotischen Zellen, den Nachweis und die automatische und schnelle Erfassung der Apoptose in einem frühen Stadium. Für die Erfassung müssen die Zellen lediglich in einem suspendierten Zustand vorliegen. Die Zugabe von einem oder mehreren Reagenzien ist nicht erforderlich. Der Aufwand bei der Probenvorbereitung ist somit sehr gering. Das Verfahren ist bezüglich der Anzahl an Zellen nicht limitiert, der Vorgang kann an einzelnen Zellen gemessen werden. Die Einzelmessung ist sehr schnell (z. B. ca. 30 Sekunden im Falle der Elektrorotation) und die Aufnahme eines zeitlichen Verlaufes an einer einzigen Zellprobe oder einer einzelnen Zelle ist möglich. Zudem hat das Verfahren keinen meßbaren Einfluß auf die Vitalität der Zellen.

Das erfindungsgemäße Verfahren zur zerstörungsfreien Bestimmung der Apoptose in einem frühen Stadium zeichnet sich dadurch aus, daß die mindestens eine zu untersuchende Zelle in ein Mikrosystem eingebracht wird, bei dem die mindestens eine Zelle hochfrequenten alternierenden, insbesondere rotierenden, elektrischen Feldern und/ oder Impedanzprüffeldern ausgesetzt wird.

Besonders bevorzugt ist die Ermittlung eines Rotationsspektrums an der Zelle durch Messung der Rotationsgeschwindigkeit (inverse Rotationszeit) der mindestens einen Zelle in Abhängigkeit von der Frequenz des rotierenden elektrischen Feldes, wobei es wünschenswert ist, daß bei Aufnahme der Rotationsgeschwindigkeit die mindestens eine Zelle gleichzeitig suspendiert im Fokus einer optischen Falle, insbesondere einer optischen Pinzette, gehalten wird. Die Rotationsmessung erfolgt vorzugsweise an Zellen, die in physiologischen Lösungen suspendiert sind. Die Leitfähigkeit der Lösung liegt vorzugsweise im Bereich von 1 ... 1.6 S/m.

Es ist allerdings nicht erforderlich, vollständige Spektren aufzunehmen. Vielmehr ist es ausreichend, die Messungen bei einer oder mehreren festgelegten Frequenzen des rotierenden Feldes vorzunehmen, vorzugsweise bei je einer Frequenz des Frequenzbereiches von 1 bis 4 MHz, vorzugsweise 2 bis 3 MHz, besonders bevorzugt 2,3 bis 2,6 MHz, und des Frequenzbereiches 5 bis 100 MHz, vorzugsweise 6 bis 50 MHz besonders bevorzugt 8 bis 15 MHz.

Zur Feststellung, ob eine untersuchte Zelle sich im Zustand der Apoptose befindet, wird die im höheren Frequenzbereich bestimmte Rotationsgeschwindigkeit zu der im niedrigeren Frequenzbereich bestimmten Rotationsgeschwindigkeit ins Verhältnis gesetzt. Ist das ermittelte Verhältnis größer als 1, besonders bevorzugt 1,1 bis 1,8, handelt es sich bei der untersuchten Zelle um eine apoptotische Zelle. Für nekrotische Zellen ist das Verhältnis kleiner 1, besonders bevorzugt 0,6 bis 0,8 und für vitale Zellen gleich 1.

Zur Bestimmung der Apoptose ist es aber auch möglich, daß das Rotationsspektrum der mindestens einen zu untersuchenden Zelle mit dem Rotationsspektrum einer Referenzzelle verglichen wird, wobei es sich bei der Referenzzelle um eine vitale, bevorzugt desselben Zelltyps wie die zu untersuchende Zelle handeln kann. Es ist aber ebenso wünschenswert, daß es sich bei der Referenzzelle um eine gezielt in den Zustand der Apoptose gebrachte Zelle, bevorzugt desselben Zelltyps wie die zu untersuchende Zelle handelt, oder daß die Referenzzelle eine gezielt in den Zustand der Nekrose gebrachte Zelle, bevorzugt desselben Zelltyps wie die zu untersuchende Zelle ist.

Der Zustand der Apoptose läßt sich dann bevorzugt dadurch feststellen, daß das Rotationsspektrum der zu untersuchenden Zelle im Gegensatz zur vitalen oder nekrotischen Referenzzelle, oder vergleichbar zur apoptotischen Referenzzelle sein Maximum im Frequenzbereich von 5 bis 100, bevorzugt 6 bis 50 MHz, besonders bevorzugt 8 bis 15 MHz aufweist.

Eine weitere Ausführung des erfindungsgemäßen Verfahrens basiert auf dem unterschiedlichen Verhalten von normalen, nekrotischen und apoptotischen Zellen in der Dielektrophorese (DEP).

Zur erfindungsgemäßen Bestimmung von Apoptose kann es daher auch wünschenswert sein, durch Messung der Wandergeschwindigkeit oder der elektrophoretischen Beweglichkeit der mindestens einen Zelle in Abhängigkeit von der Frequenz des elektrischen Feldes ein Dielektrophoresespektrum bevorzugt im Frequenzbereich von 0 bis 5 MHz, besonders bevorzugt von 1 bis 4 MHz zu ermitteln. Wobei in einer speziellen Ausführungsform die mindestens eine Zelle im Fokus einer optischen Falle gehalten wird. Erfindungsgemäß wird Apoptose dadurch festgestellt, daß der Frequenzdurchtritt des Überganges von negativer zu positiver Dielektrophorese im Dielektrophoresespektrum im Bereich von 3,3 bis 3,8 MHz, bevorzugt bei 3,5 MHz liegt.

Liegt der Frequenzdurchtritt des Überganges von negativer zu positiver Dielektrophorese bei 2,8 bis 3,1 MHz, bevorzugt bei 3 MHz, so handelt es sich bei der untersuchten Zelle um eine vitale Zelle, liegt er bei 1,8 bis 2,2 MHz, bevorzugt bei 2 MHz, so liegt eine nekrotische Zelle vor.

Apoptose läßt sich auch dadurch bestimmen, daß das Dielektrophoresespektrum der mindestens einen zu untersuchenden Zelle mit dem Dielektrophoresespektrum einer Referenzzelle verglichen wird, wobei es sich bei der Referenzzelle um eine vitale, bevorzugt desselben Zelltyps wie die zu untersuchende Zelle handeln kann. Es ist aber ebenso wünschenswert, daß es sich bei der Referenzzelle um eine gezielt in den Zustand der Apoptose gebrachte Zelle, bevorzugt desselben Zelltyps wie die zu untersuchende Zelle handelt, oder daß die Referenzzelle eine gezielt in den Zustand der Nekrose gebrachte Zelle, bevorzugt desselben Zelltyps wie die zu untersuchende Zelle ist.

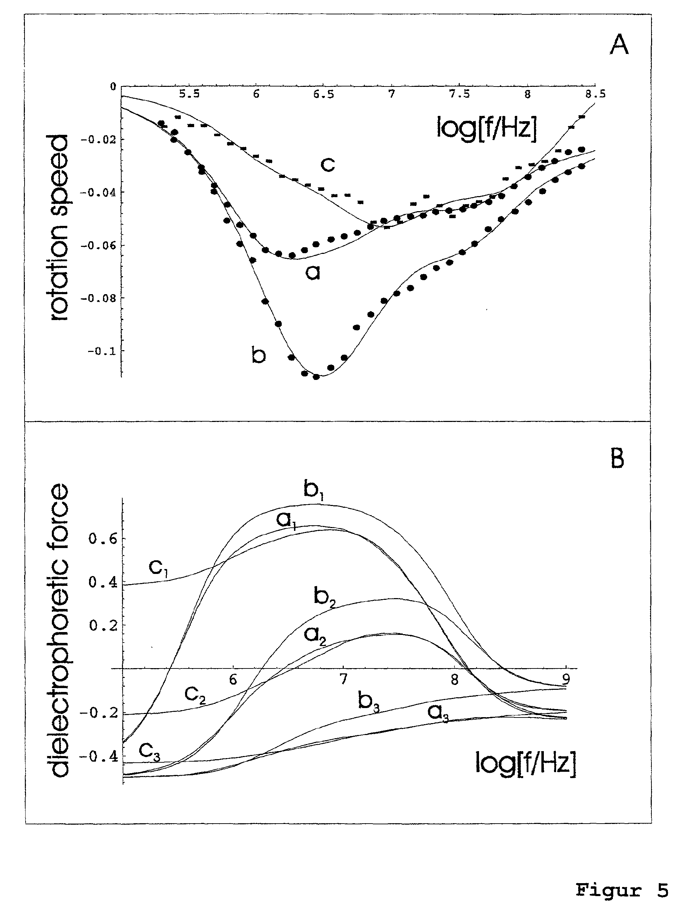

In einer weiteren Ausführungsform des erfindungsgemäßen Verfahrens wird die unterschiedliche Kraftwirkung, die durch ein elektrisches Wechselfeld auf vitale, apoptotische und nekrotische Zellen ausgeübt wird, ausgenutzt, um die verschiedenen Zelltypen zu trennen. Die Erhöhung der Innenleitfähigkeit und der Dielektrizitätskonstante von nekrotischen Zellen gegenüber vitalen Zellen, führt zu veränderten Werten des Realteiles des elektrischen Dipolmomentes. In PBS-Puffer ist die Kraftwirkung auf nekrotische Zellen gegenüber vitalen und apoptotischen Zellen bevorzugt im Frequenzbereich über 1 MHz deutlich verringert (Abbildung 5) und so eine dielektrische Separation möglich.

Eine Absenkung des Elektrolytgehaltes der Pufferlösung unter einen Bereich um 0.3 S/m bedingt vorzugsweise im Frequenzbereich bis 3 MHz für apoptotische und nekrotische Zellen eine geringere negative DEP im Vergleich zu vitalen Zellen. In einem Frequenzbereich von 3 bis 120 MHz kommt es dem gegenüber zu stark abgeschwächter positiver DEP von apoptotischen und normalen Zellen im Vergleich zu nekrotischen Zellen. Der Frequenzdurchtritt des Überganges von negativer zu positiver DEP liegt für apoptotische Zellen hier im Bereich von 3,3 bis 3,8 MHz, bevorzugt bei 3,5 MHz, für vitale Zellen im Bereich von 2,8 bis 3,1 MHz, bevorzugt bei 3 MHz und für die nekrotischen Zellen im Bereich von 1,8 bis 2,2 MHz, bevorzugt bei 2 MHz. Somit ist eine effektive Trennung aller drei Zelltypen durch dielektrophoretische Kräfte möglich. So lassen sich beispielsweise die Anteile von nekrotischen, apoptotischen und vitalen Zellen einer Probe ermitteln (Abbildung 5B).

Entsprechend einer weiteren Ausführungsform des erfindungsgemäßen Verfahrens, kann die Bestimmung der Apoptose auch durch Messung der Impedanzeigenschaft (Betrag und/oder Phase) der mindestens einen Zelle in Abhängigkeit von der Frequenz des Impedanzprüffeldes erfolgen. Es wird ein Impedanzspektrum ermittelt, wobei vorzugsweise die mindestens eine Zelle im Fokus einer optischen Falle gehalten wird. Ebenfalls wünschenswert ist, daß die Impedanz einzelner Zellen zwischen Mikroelektroden in kleinen Volumina bestimmt wird. Die Impedanz einer Zellsuspension berechnet sich zu:

$$Z(\omega) \; = \; \cfrac{1}{\left( \cfrac{1}{R_{ext}} \; + \; \cfrac{1}{R_C \; + \; 1\big/i\omega C_M} \right)}$$

(Fricke, H., 1923, The electric capacity of cell suspensions, Phys. Rev. 21, 708-709), wobei $R_{ext}$ den externen Lösungswiderstand, $R_c$ den Zytoplasmawiderstand und $C_M$ die Membrankapazität darstellen. Für eine genaue Analyse biologischer Zellen sind komplexe Modelle nötig, die die Kapazität und Ohm schen Anteile aller Kompartimente widerspiegeln (Irimajiri, A., Suzaki, T., Asami, K. und Hanai, T., 1991, Dielectric Modeling of Biological Cells. Models and Algorithm, Bull. Inst. Chem. Res. Kyoto Univ. 69/4, S. 421-438).

Um erfindungsgemäß eine hohe Empfindlichkeit der Messung zu gewährleisten, ist es wünschenswert, daß der Beitrag der Lösung 1/Rext klein gegenüber dem Beitrag der einzelnen Zelle ist. Dies gilt entweder für kleine Frequenzen oder für einen geringen Abstand zwischen der Zelle und den ebenfalls kleinen Messelektroden (Einzelzellimpedanzmessung), wobei die Einzelimpedanzmessung zur Bestimmung von Differenzen der Zytoplasmaeigenschaften im Megahertz-Bereich besonders wünschenswert ist. Zusätzlich führen die Zellveränderungen während der Apoptose zu einem veränderten Impedanzsignal im niederfrequenten Bereich: 1 Hz - 100 kHz, bevorzugt 0,5 kHz - 10 kHz. Zur Bestimmung der Apoptose ist es daher bevorzugt Impedanzspektren in diesem Frequenzbereich aufzunehmen.

Zur Bestimmung der Apoptose ist es auch möglich, daß das Impedanzspektrum der mindestens einen zu untersuchenden Zelle mit dem einer Referenzzelle verglichen wird, wobei es sich bei der Referenzzelle um eine vitale, bevorzugt desselben Zelltyps wie die zu untersuchende Zelle handeln kann. Es ist aber ebenso wünschenswert, daß es sich bei der Referenzzelle um eine gezielt in den Zustand der Apoptose gebrachte Zelle, bevorzugt desselben Zelltyps wie die zu untersuchende Zelle handelt, oder daß die Referenzzelle eine gezielt in den Zustand der Nekrose gebrachte Zelle, bevorzugt desselben Zelltyps wie die zu untersuchende Zelle ist.

Der Zustand der Apotose läßt sich dann bevorzugt dadurch feststellen, daß das Impedanzspektrum der zu untersuchenden Zelle im Gegensatz zur vitalen oder nekrotischen Referenzzelle, oder vergleichbar zur apoptotischen Referenzzelle insbesondere im Frequenzbereich von 1 Hz - 100 kHz, bevorzugt 0,5 kHz - 10 kHz Änderungen aufweist.

Zur Verfolgung der zeitlichen Änderung der Eigenschaften der mindestens einen Zelle, insbesondere zur Bestimmung des zeitlichen Verlaufs der Apoptose, kann es wünschenswert sein, daß die Rotationsspektren und/oder die mindestens 2 Rotationsgeschwindigkeiten und/oder die Dielektrophoresespektren und/oder die Impedanzspektren in Abhängigkeit von der Zeit aufgenommen werden.

Insgesamt eignet sich das erfindungsgemäße Verfahren besonders zur Diagnose und/oder Therapiekontrolle von Krankheiten und/oder Prozessen, die mit einer Erhöhung der Apoptoserate verbunden sind, wie insbesondere AIDS, neurodegenerative Erkrankungen, insbesondere Alzheimer, Parkinson; durch Gifte, insbesondere Alkohol, verursachte Leberkrankheiten; Krankheiten, die eine zu geringe Hormonproduktion oder -ausschüttung hervorrufen, eingesetzt werden.

Erfindungsgemäß ist es auch möglich, das Verfahren zur Bestimmung von Apoptose zur Diagnose und/oder Therapiekontrolle von Krankheiten und/oder Prozessen die mit einer Erniedrigung der Apoptoserate verbunden sind, wie insbesondere maligne und benigne hyperproliferative Erkrankungen, wie Krebs, hormon-abhängige Tumore, Leukämie; Autoimmunkrankheiten, insbesondere Arthritis, Diabetis mellitus und virale Infektionen, insbesondere durch Herpesviren, Poxviren oder Adenoviren hervorgerufene Krankheiten, einzusetzen.

Das erfindungsgemäße Verfahren ermöglicht ferner, insbesondere aufgrund seiner schnellen Durchführbarkeit und auch der geringen benötigten Mengen zu untersuchender Zellen. Das Verfahren kann sogar an einer einzelnen Zelle durchgeführt werden. Es ist somit sehr zu gut zur Therapiekontrolle von insbesondere malignen und benignen hyperproliferativen Erkrankungen bei der Durchführung einer Chemotherapie, Radiotherapie, Immuntherapie, Operationen oder einer Kombination dieser Therapien, geeignet.

Erfindungsgemäß ist es auch gelungen ein Verfahren bereitzustellen, daß die Identifizierung von Apoptosebeeinflussenden Substanzen unter Berücksichtigung der zellulären Rezeptoren und anderer Kofaktoren erlaubt und so die Gefahr der Identifizierung von falsch-positiven oder falsch-negativen Apoptose-beeinflussenden Substanzen herabsetzt.

Ein erfindungsgemäße Verfahren zur Identifizierung von Apoptose-beeinflussenden Substanzen ist insbesondere durch die folgenden Schritte gekennzeichnet:

- Bereitstellen mindestens einer zu untersuchenden Zellkulturprobe

- Zugabe einer potentiellen Apoptose beeinflussenden Substanz oder Mischungen aus mindestens zwei dieser Substanzen,
- Einbringen mindestens einer Zelle der zu untersuchenden Zellkulturprobe in ein Mikrosystem, bei dem die mindestens eine Zelle hochfrequenten alternierenden, insbesondere rotierenden, elektrischen Feldern und/oder Impedanzprüffeldern ausgesetzt wird,
- Bestimmung der Apoptose-beeinflussenden Eigenschaften durch Vergleich des Verhaltens dieser mindestens einen Zelle im elektrischen Feld mit dem mindestens einer Zelle einer Referenzprobe.

Bei der Referenzprobe ist es bevorzugt, eine Probe desselben Zelltyps wie die zu untersuchende Probe, vor Zugabe der mindestens einen potentiellen Apoptose-beeinflussenden Substanz, zu verwenden.

In einer weiteren Ausführungsform des erfindungsgemäßen Verfahrens wird das Verhalten der mindestens einen Zelle der zu untersuchenden Zellkulturprobe und der mindestens einen Zelle der Referenzprobe im elektrischen Feld durch Aufnahme eines Rotationsspektrums durch Messung der Rotationsgeschwindigkeit jeder Zelle in Abhängigkeit von der Frequenz der rotierenden elektrischen Felder untersucht.

Die Apoptose-beeinflussenden Eigenschaften der mindestens einen Substanz lassen sich erfindungsgemäß dadurch feststellen, daß das Rotationsspektrum der mindestens einen zu untersuchenden Zelle der Probe im Gegensatz zum Spektrum der mindestens einen Zelle der Referenzprobe eine Änderung insbesondere im Frequenzbereich von 5 bis 100, bevorzugt 6 bis 50 MHz, besonders bevorzugt 8 bis 15 MHz und/oder im Frequenzbereich von 1-4 MHz, bevorzugt 2 bis 3 MHz, besonders bevorzugt 2,3-2,6 MHz Bereich aufweist.

Es kann aber auch bevorzugt sein, daß die Rotationsgeschwindigkeit der Zellen der Zellprobe und der Referenz nur bei mindestens je einer Frequenz des Frequenzbereiches 1 bis 4 MHz, vorzugsweise 2 bis 3 MHz, besonders bevorzugt 2,3 bis 2,5 MHz und des Frequenzbereiches 5 bis 100 MHz, vorzugsweise 6 bis 50 MHz besonders bevorzugt 8 bis 15 MHz bestimmt wird, wobei das die im höheren Frequenzbereich bestimmte Rotationsgeschwindigkeit zu der im niedrigeren Frequenzbereich bestimmten Rotationsgeschwindigkeit ins Verhältnis gesetzt wird.

Für eine apoptotische Zelle ist das Verhältnis größer als 1, besonders bevorzugt 1,1 bis 1,8; für eine nekrotische Zelle kleiner 1, besonders bevorzugt 0,6 bis 0,8 und für eine vitale Zelle gleich 1.

Es kann aber ebenfalls wünschenswert sein, das Verhalten der mindestens einen Zelle der zu untersuchenden Zellkulturprobe und der mindestens einen Zelle der Referenzprobe im elektrischen Feld durch Aufnahme eines Dielektrophoresespektrums durch Messung der Wandergeschwindigkeit oder dielektrischen Beweglichkeit jeder Zelle in Abhängigkeit von der Frequenz der alternierenden elektrischen Felder zu untersuchen.

In einer weiteren Ausführungsform des Verfahrens werden die Apoptose-beeinflussenden Eigenschaften der mindestens einen Substanz dadurch festgestellt, daß im Dielektrophoresespektrum der mindestens einen zu untersuchenden Zelle im Gegensatz zum Spektrum der mindestens einen Zelle der Referenzprobe der Frequenzdurchtritt des Überganges von negativer zu positiver Dielektrophorese im Bereich von 3,3 bis 3,8 MHz, bevorzugt bei 3,5 MHz liegt, wobei das Dielektrophoresespektrum der mindestens einen zu untersuchenden Zelle der Probe im Frequenzbereich von 0 bis 5 MHz bevorzugt 1 bis 4 MHz aufgezeichnet wird.

In einer weiteren Ausführungsform des erfindungsgemäßen Verfahrens wird das Verhalten der mindestens einen Zelle der zu untersuchenden Zellkulturprobe und der mindestens einen Zelle der Referenzprobe im elektrischen Feld durch Aufnahme eines Impedanzspektrums durch Messung der Impedanzeigenschaften (Betrag und/oder Phase) jeder Zelle in Abhängigkeit von der Frequenz des Impedanzprüffeldes untersucht.

Eine weitere Ausführungsform des Verfahrens beschreibt, daß die Apoptose-beeinflussenden Eigenschaften der mindestens einen Substanz dadurch festgestellt werden, daß im Impedanzspektrum der mindestens einen zu untersuchenden Zelle im Gegensatz zum Spektrum der mindestens einen Zelle der Referenzprobe Änderungen im Frequenzbereich von 1 Hz bis 100 kHz, bevorzugt 0,5 kHz - 10 kHz, auftreten.

Bei Aufnahme des Rotationsspektrums bei mindestens einer Frequenz und/oder eines Dielektrophoresespektrums und/oder eines Impedanzspektrums kann es wünschenswert sein, das die mindestens eine Zelle im Fokus einer optischen Falle gehalten wird.

Zur Bestimmung des Einflusses von potentiellen Apoptose-beeinflussenden Substanzen kann es bevorzugt sein, den Anteils an apoptotischen Zellen in einer Probe zu bestimmen. Hierzu kann zunächst eine dielektrische Separation der Zellen eines bestimmten Volumens der Probe und/oder der eines bestimmten Volumens der Referenzprobe durchgeführt werden. Anschließend wird die Anzahl der apoptotischen Zellen der zu untersuchenden Probe im Verhältnis zu der Anzahl der apoptotischen Zellen der Referenzprobe ermittelt.

Apoptose-erhöhende Substanzen zeichnen sich demnach durch eine Erhöhung der Anzahl apoptotischer Zellen im Verhältnis zur Referenzprobe aus.

Substanzen, die die Apoptose nicht beeinflussen, sind durch einen zur Referenzprobe vergleichbaren Anteil apoptotischer Zellen charakterisiert.

Apoptose-hemmende Substanzen zeichnen sich demnach durch eine geringere Anzahl apoptotischer Zellen im Verhältnis zur Referenzprobe aus.

Da die natürliche Apoptoserate in Zellkulturen häufig sehr gering ist, kann es bei der Bestimmung von potentiellen Apoptose-hemmenden Substanzen auch wünschenswert sein, vor oder während der Zugabe der mindestens einen zu testenden Substanz, die zu untersuchende Zellkulturprobe mit einer Apoptose-erhöhenden Substanz zu versetzen, wobei als Referenzprobe die mit einer Apoptose-erhöhenden Substanz versetzte Zellkulturprobe und oder eine Zellkulturprobe, die dengleichen Anteil Apoptose-erhöhende Substanz erhält, eingesetzt wird.

Eine Apoptose-erniedrigende Substanz zeichnet sich nun dadurch aus, daß sie im Verhältnis zur Referenzprobe einen geringeren Anteil apoptotischer Zellen aufweist.

Insgesamt ermöglicht die Bestimmung von Apoptose gemäß der vorliegenden Erfindung, Apoptose mit einem einfachen, effizienten, schnellen und hochdurchsatzfähigen Verfahren durch Erkennung früher Stadien der Apoptose ohne Zerstörung der Zelle zu ermitteln. Ein weiterer Vorteil des erfindungsgemäßen Verfahrens ist, daß dieses Verfahren auf Zellen in ihrer physiologischen Umgebung angewendet werden kann ohne die Eigenschaften der Zelle oder ihrer Oberfläche zu ändern. Die Verfahren zur Bestimmung von Apoptose-beeinflussenden Substanzen sind dazu in der Lage, exakt deren Fähigkeit, in die Zelle einzudringen, unter Berücksichtigung des Einflusses von Rezeptoren und Kofaktoren, zu bestimmen. Die Gefahr einer falsch-positiven oder falsch-negativen Ermittlung von Apoptose-beeinflussenden Substanzen ist deutlich geringer als bei den bekannten Methoden. Zudem ist es möglich, Zelltyp- oder Organspezifische Modulatoren zu ermitteln. Darüber hinaus sind zur Durchführung des erfindungsgemäßen Verfahrens keine spezifischen, die Zellwand durchdringenden Fluoreszenzfarbstoffe notwendig.

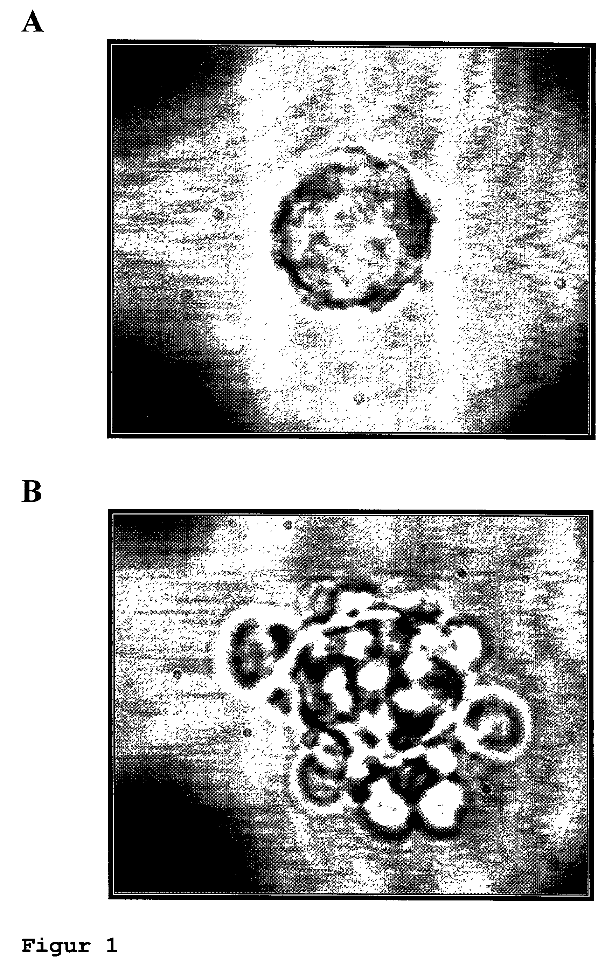

Figur 1 zeigt lichtmikroskopische Aufnahmen einer normalen (A) und einer apoptotischen (B) Jurkat-Zelle (Klon E6.1 von der European Collection of Animal Cell Cultures, Salisbury, England). Die apoptotische Zelle wurde für 4 Stunden mit 5 μg/ml Actinomycin D behandelt.

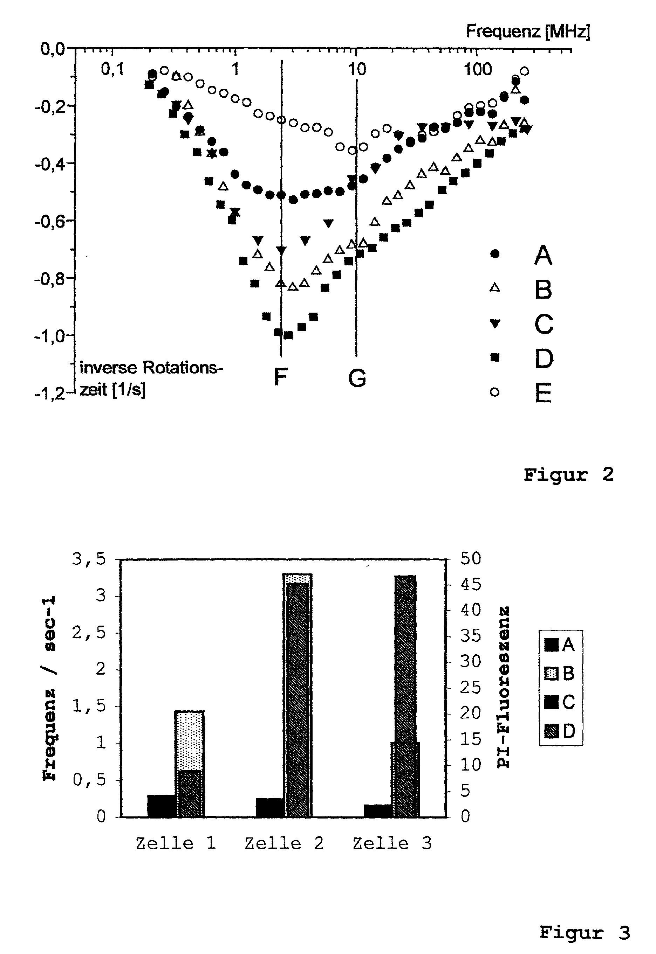

Figur 2 zeigt Elektrorotationsspektren einer normalen Jurkat-Zelle (A), einer für ca. 5 Minuten mit 10 μm Ionomycin-behandelten Jurkat-Zelle (B), einer für 20 Sekunden mit 50 kHz und Erms = 30 kV/m dielektrophoretisch geschädigten Jurkat-Zelle (C), einer für 40 Minuten mit 30 mW einer optischen Pinzette von 1080 nm Wellenlänge gehaltenen RMA-Zelle (Thymoma - Zellinie von dem Klon C57BL/6 von Professor Dr. Peter Walden, Charité, Berlin) (D) und einer für 4 Stunden mit Actinomycin D-behandelten Jurkat-Zelle (E).

In Figur 3 ist die Beschleunigung der Elektrorotation und Aufnahme von Propidiumiodid bei nekrotischer Schädigung von Jurkat-Zellen durch Erhöhung der Spannung in einem dielektrophoretischen Oktupol-Feldkäfig dargestellt. (A) Rotationsgeschwindigkeit (inverse Rotationszeit) einer Jurkat-Zelle bei 4,3 V, (B) Frequenz der Rotation derselben Jurkat-Zelle nach Erhöhen der Spannung auf 6,8 V. (C) Propidiumiodid-Fluoreszenz der Jurkat-Zelle bei 4,3 V, (D) Propidiumiodid-Fluoreszenz derselben Jurkat-Zelle nach Erhöhen der Spannung auf 6,8 V. Die Elektrorotation wurde jeweils mit 4,3 V gemessen. Drei verschiedene Zellen wurden untersucht.

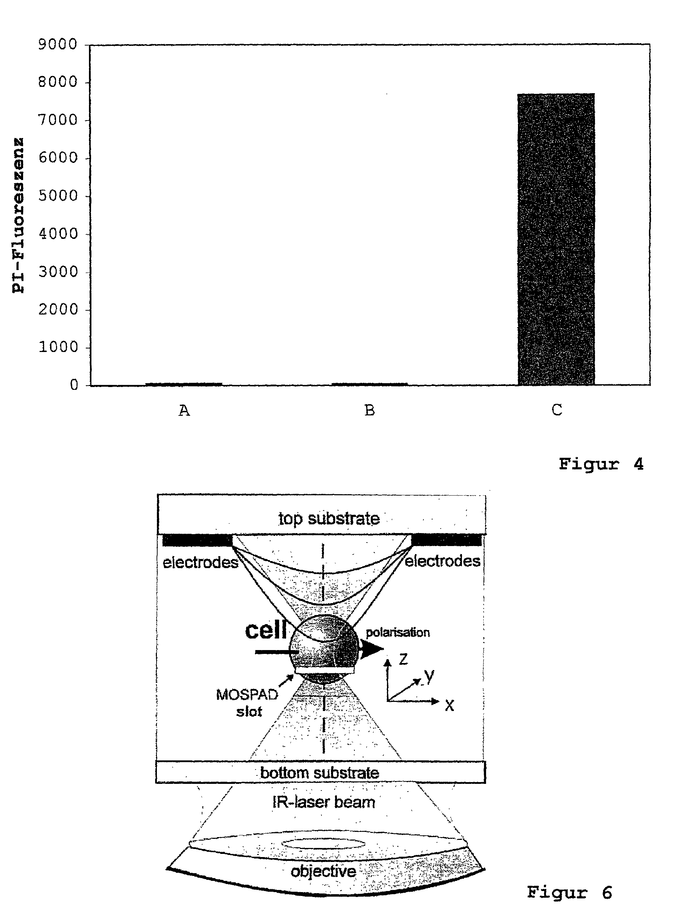

Figur 4 zeigt die Propidiumiodid-Fluoreszenz einer normalen U937-Zelle (A), einer für 4 Stunden mit Actinomycin D-behandelten, apoptotischen U937-Zelle (Monozytische Zellinie von der European Collection of Animal Cell Cultures, Salisbury, England) (B) und einer nekrotischen U937-Zelle (C).

In Figur 5 sind Elektrorotation und Dielektrophorese von T-Lymphozyten (Jurkat) in Abhängigkeit von der Feldfrequenz dargestellt. Die Kurven a,b und c gehören zu normalen, nekrotischen und apoptotischen Zellen. In A sind automatische Messungen der Elektrorotation in PBS (Leitfähigkeit 1.5 S/m) sowie Kurvenfits entsprechend mehrschaliger sphärischer Modelle mit Dispersion gezeigt. B zeigt das dazu korrespondierende dielektrophoretische Verhalten wobei die Indizes der Kurven zu verschiedenen externen Leitfähigkeiten korrespondieren (1: 0.05 S/m, 2: 0.3 S/m, 3: 1.5 S/m (PBS)).

Das dispersive Materialverhalten wird als Summe von Relaxationen beschrieben (Dielektrizitätskonstante ε, Leitfähigkeit σ, Kreisfrequenz des elektrischen Feldes ω, Relaxationszeit der Dispersion τ, Anzahl der Dispersionen n):

$$\varepsilon = \varepsilon_\infty + \frac{1}{\varepsilon_{vac}} \sum_{k=1}^{n} \frac{\tau_k \Delta\sigma_k}{1 + (\omega\tau_k)^2} \quad mit \quad \Delta\varepsilon = -\tau\Delta\sigma$$

Figur 6 zeigt einen seitlichen Blick auf ein Mikrosystem für Elektrorotationsmessungen in Mikrokanälen (C. C. Reichle, T. Schnelle, T. Müller, T. Leya und G. Fuhr, 2000, A new microsystem for automated electrorotation measurements using laser tweezers, Biochimica et Biophysica Acta 1459, S. 218-229).

**BEISPIEL 1**

**Elektrorotationsmessung**

Verwendete Materialien:

| | |
|---|---|
| Jurkat Zellen: | Klon E6.1 von der European Collection of Animal Cell Cultures, Salisbury, England; |
| U937 Zellen: | Monozytische Zellinie von der European Collection of Animal Cell Cultures, Salisbury, England; |
| RMA Zellen: | Thymoma - Zellinie von dem Klon C57BL/6 von Professor Dr. Peter Walden, Charité, Berlin, Ref: C. Reichle, K. Sparbier, T. Müller, T. Schnelle, P. Walden und G. Fuhr, in press, Combined laser tweezers and dielectric field cage for the analysis of multivalent receptor ligand interactions on single cells, ELECTROPHORESIS, Miniaturization II; |
| Actinomycin D: | SIGMA Aldrich GmbH, Steinheim, Deutschland; |
| Ionomycin: | Calbiochem, Bad Soden, Deutschland; |
| Propidiumiodid: | SIGMA Aldrich GmbH, Steinheim, Deutschland; |
| PBS: | Phosphate Buffered Saline, mit Calcium und Magnesium, Seromed / Biochrom, Berlin, Deutschland; |
| RPMI 1640 Medium: | HEPES buffered, GIBCO Life Technologies, Karlsruhe, Deutschland; |
| Fötales Kälberserum: | Seromed / Biochrom, Berlin, Deutschland; |
| Penicillin: | Seromed / Biochrom, Berlin, Deutschland; |
| Streptomycin: | Seromed / Biochrom, Berlin, Deutschland; |
| Inosit: | SIGMA Aldrich GmbH, Steinheim, Deutschland. |

Vorrichtung:

Es wurde eine bereits von C. C. Reichle, T. Schnelle, T. Müller, T. Leya und G. Fuhr ( 2000, A new microsystem for automated electrorotation measurements using laser tweezers, Biochimica et Biophysica Acta 1459, S. 218-229.) beschriebene Vorrichtung, eine Kombination aus dieelektrophoretischen Feldkäfigen mit optischen Fallen, insbesondere Laser-Pinzetten, zur Bestimmung der Elektrorotationsspektren eingesetzt (Figur 6). Eine vergleichbare Vorrichtung ist auch in T. Schnelle, T. Müller, C. Reichle und G. Fuhr 2000, Combined dielectrophoretic field cages and laser tweezers for electrorotation, Applied Physics B, Lasers and Optics, Springer-Verlag beschrieben.

Durchführung:

Jurkat T-Lymphomzellen und U937 monozytische Zellen wurden in RPMI-1640 Medium mit Zusatz von 10% fötalem Kälberserum und 100 IU/ml Penicillin sowie 100 IU/ml Streptomycin kultiviert. Apoptose wurde durch Zugabe von 5 µg/ml Actinomycin D in einer am Vortag mit $2 \times 10^5$ Zellen pro ml ausgesäten Kultur ausgelöst. Die klassischen morphologischen Veränderungen während der Apoptose wurden fotografisch festgehalten (siehe Figur 1). Zu verschiedenen Zeitpunkten wurde ein Aliquot der Kultur entnommen, in einer Mischung aus isotonischem Phosphatpuffer (PBS, D8662 von SIGMA mit Calcium und Magnesium) und wahlweise 0.3 M Inosit mit der jeweils angegebenen elektrischen Leitfähigkeit aufgenommen und in eine Vorrichtung zur Messung der Elektrorotation in einem Mikro-Elektrodensystem eingeführt. Als Kontrolle wurden unbehandelte Jurkat-Zellen verwendet (Figur 5). Zum Vergleich wurden Elektrorotationsspektren (ER-Spektren) von Ionomycin-behandelten Zellen (10 µg/ml Ionomycin) sowie von Zellen, deren Plasmamembran durch eine Erhöhung der Spannung von 4 V auf 6,8 V oder eine Behandlung mit einer optischen Pinzette mit 30-60 mW (2.8 A des 1 W Nd:YAG-Laser, $TEM_{00}$, 1064nm, LD 3000i, Laser Quantum Ltd., Machester, UK, P.A.L. M. System) über 15 Minuten geschädigt wurde. Die Zellen wurden hierzu einzeln in das Zentrum des Quadrupols bzw. Oktupols des Mikro-Elektrodensystems überführt. Hierzu wurde in den angezeigten Fällen eine optische Pinzette mit ca. 30 mW Leistung am Ort des Haltens zu Hilfe genommen. Die Frequenz der Zellrotation wurde entweder automatisch oder mit Hilfe einer Stoppuhr gemessen (Figur 2). Die Aufnahme von Propidiumiodid (5 µM) in nekrotisch geschädigte Zellen bzw. Vergleichsmessungen mit vitalen und apoptotischen Zellen (Figuren 3 und 4) wurden durch konfokale Nanofluorimetrie (Gradl, T. Müller, C. Reichle, T. Schnelle und G. Fuhr, 1999, Micro-Electrode Systems for Cell Analysis, European Journal of Cell Biology, Suppl. 49, Vol. 78; T. Schnelle, T. Müller, G. Gradl, S. G. Shirley und G. Fuhr, 2000, Dielectrophoretic manipulation of suspended submicron particles, ELECTROPHORESIS 2000, 21, S. 66-73) gemessen.

Elektrorotationsmessung:

Die Vorrichtung zur Messung der Elektrorotation wurde auf einem Fluoreszenzkorrelationsspektrometer aufgebaut. Die Zellen wurden über ein 40 x W 1.2 NA Objektiv abgebildet. Sie wurden mit einem Helium-Neon-Laser von 543 nm Wellenlänge mit 1,5 µW angeregt und die ausgestrahlte Fluoreszenz durch einen 585 DF 35 Bandpaßfilter über ein 50 um großes Pinhole auf eine Photodiode geleitet, mit der die Fluoreszenz-Photonen gezählt wurden.

Ergebnis:

Das charakteristische ER-Spektrum einer in Figur 1 gezeigten normalen (A) Jurkat-Zelle zeigt einen breiten Peak von 1,5 bis 7 MHz, bei der eine maximale Rotationsgeschwindigkeit der Zelle gemessen wird, mit einem Maximum um 2,5 MHz (Figur 2 A).

Eine mit dem Ionophor Ionomycin (Calbiochem, Bad Soden, Deutschland)behandelten Zelle unterscheidet sich deutlich im ER-Spektrum (Figur 2 B) von einer normalen Zelle. Ionomycin verursacht in höherer Konzentration eine Aufnahme von Calcium aus dem extrazellulären Raum in die Zelle (J. B. Smith, T. Zheng und R.-M. Lyu, 1989, Ionomycin releases calcium from the sarcoplasmatic reticulum and activates $Na^+/Ca^{2+}$ exchange in vascular smooth muscle cells, Cell Calcium 10, S. 125-134). Bei der Ionomycin Konzentration von 10 µM zeigen die Fits zu den zugehörigen ER-Spektren, daß zusätzlich eine Aufnahme von Wasser und vermutlich auch von Pufferionen in substanzieller Menge, wie Natrium und Kalium, stattfindet. Dies ist anhand der Zunahme der Leitfähigkeit σ (als Summe von $\sigma_0$, $\Delta\sigma_1$ und $\Delta\sigma_2$) von 0,77 auf 1,15 S $m^{-1}$ und der Dielektrizitätskonstante $\varepsilon_\infty$ von 45 auf 75 innerhalb der Zelle deutlich zu erkennen (C. Reichle, T. Schnelle, T. Müller, T. Leya und G. Fuhr, 2000, A new microsystem for automated electrorotation measurements using laser tweezers, Biochimica et Biophysica Acta 1459, S. 218-229). Sie nähern sich den Werten des umgebenden Puffers PBS an.

Da die dielektrisch wirksame Gesamtkonzentration einer Zelle an den Ionen Natrium und Kalium vor der Behandlung niedriger ist im Vergleich zur Ionenkonzentration des PBS (Phosphate Buffered Saline, mit Calcium und Magnesium, Seromed / Biochrom, Berlin, Deutschland); ca. 0.54 S/m zu 1.5 S/m; erfolgt ein Netto-Einfluß an Ionen und Wasser und die Zelle schwillt an. Im ER-Spektrum schlägt sich dies in einer starken Vergrößerung des 2,5 MHz Peaks nieder und ist mit einer ca. 40%-igen Erhöhung der HF-Leitfähigkeit und einem über 60% igem Anstieg in der Dielektrizitätskonstante des Zytoplasmas erklärbar. Ein ganz ähnliches Verhalten zeigen Zellen, die eine nekrotische Schädigung durch das Anlegen einer erhöhten Spannung im dielektrischen Feldkäfig erfahren haben (Figur 2 C). Dieser dielektrische Durchbruch ist mit einer Schädigung der Plasmamembran verbunden, was durch die Aufnahme des DNA-Farbstoffes Propidiumiodid sichtbar gemacht werden kann (Figur 3). Daraufhin können sich Ionen hier ebenfalls zwischen Zellinnenraum und umgebendem Puffer austauschen. Das sich ergebende ER-Spektrum ist dem der Ionomycin-behandelten Zelle sehr ähnlich. Auch eine nekrotische Schädigung mit einer optischen Pinzette führt zu der gleichen Erscheinung (Figur 2 D). Alle bis hierhin beschriebenen Schädigungen sind mit einem Verlust der Integrität der Plasmamembran und einem Angleich des Ionengehaltes von Zellinnenraum und Außenraum verbunden. Bei Absenken der Leitfähigkeit der Außenlösung unter die der Zelle, würde ein Nettoefflux an Ionen den Elektrolytgehalt der Zelle verringern. Dies führt zu einem weiteren Absinken der Frequenz mit der schnellsten Rotation (fc) und verstärkt die Unterschiede zu den unbehandelten Zellen. Überraschenderweise unterscheidet sich ein charakteristisches Spektrum einer apoptotischen Zelle von dem einer normalen Zellen bzw. dem einer nekrotisch geschädigten Zelle (Figur 2E). Der Peak bei 2,5 MHz verschwindet völlig. Die höchste Rotationsgeschwindigkeit ist bei etwa 10 MHz zu verzeichnen. Eine typische apoptotische Zelle mit diesem beschriebenen ER-Spektrum weist starke Plasmamembranprotuberanzen auf (s. Figur 1B). In diesem Stadium ist ihre Plasmamembran jedoch immer noch intakt (Figur 4). Somit ist das erfindungsgemäße Verfahren geeignet, zwischen normalen, apoptotischen und nekrotisch geschädigten Zellen zu unterscheiden.

Elektrorotationsmessungen bei den beiden Frequenzen 2,5 MHz (Figur 2 F) und 10 MHz (Figur 2G) können den Unterschied zwischen diesen drei unterschiedlichen Zellstadien bereits deutlich erkennbar machen. Das Verhältnis der Rotationsgeschwindigkeit (inversen Rotationszeit) der Messung bei 10 MHz und der Messung bei 2,5 MHz ist für die normale Zelle nahe bei 1 (mit etwa 10% Abweichung). Für die nekrotisch geschädigte Zelle ist es deutlich kleiner als 1 (0,6 - 0,8) und für die apoptotische Zelle ist es größer als 1 (in diesem Fall 1,4).

**Patentansprüche**

1. Verfahren zur zerstörungsfreien Vitalitätsmessung an biologischen Zellen, insbesondere zur Bestimmung von Apoptose, **dadurch gekennzeichnet, dass** mindestens eine Zelle hochfrequenten alternierenden, insbesondere rotierenden, elektrischen Feldern und/oder Impedanzprüffeldern ausgesetzt wird und an der Zelle für mindestens einen Frequenzbereich oder einzelne Frequenzen mindestens eine Rotationsmessung, eine Dielektrophoresemessung und/oder eine Impedanzmessung erfolgen, aus denen mindestens ein Messparameter ermittelt wird, der für den Vitalitätszustand der Zelle charakteristisch ist.

2. Verfahren gemäß Anspruch 1, bei dem mindestens ein Rotationsspektrum der mindestens einen Zelle durch Erfassung der Rotationsgeschwindigkeit der Zelle in Abhängigkeit von der Frequenz des rotierenden elektrischen Feldes gemessen wird.

3. Verfahren gemäß mindestens einem der Ansprüche 1 und 2, bei dem die Rotationsgeschwindigkeit der mindestens einen Zelle bei mindestens je einer Frequenz des Frequenzbereiches 1 bis 4 MHz, vorzugsweise 2 bis 3 MHz, besonders bevorzugt 2,3 bis 2,6 MHz und des Frequenzbereiches 5 bis 100 MHz, vorzugsweise 6 bis 50 MHz besonders bevorzugt 8 bis 15 MHz bestimmt wird.

4. Verfahren gemäß Anspruch 3, bei dem als Messparameter der Quotient aus den Rotationsgeschwindigkeiten ermittelt wird, die jeweils im höheren und im niedrigeren Frequenzbereich auftreten.

5. Verfahren gemäß Anspruch 4, bei dem eine apoptotische Zelle dadurch bestimmt wird, daß der Quotient größer als 1, besonders bevorzugt 1,1 bis 1,8, ist, wobei der Quotient für nekrotische Zellen kleiner 1, besonders bevorzugt 0,6 bis 0,8 und für vitale Zellen gleich 1 ist.

6. Verfahren gemäß mindestens einem der Ansprüche 1 bis 5, bei dem Apoptose dadurch bestimmt wird, daß das Rotationsspektrum und oder die mindestens 2 Rotationsgeschwindigkeiten der mindestens einen zu untersuchenden Zelle mit dem Rotationsspektrum und/oder den mindestens 2 bei denselben Frequenzen, wie der zu untersuchenden Zelle ermittelten Rotationsgeschwindigkeiten einer Referenzzelle, verglichen wird/werden.

7. Verfahren gemäß Anspruch 6, bei dem Apoptose dadurch festgestellt wird, dass das Rotationssprektrum der zu untersuchenden Zelle im Gegensatz zur Referenzzelle sein Maximum in einem niedrigeren Frequenzbereich aufweist, wobei die Referenzzelle eine native oder eine gezielt in den Zustand der Nekrose gebrachte Zelle, bevorzugt desselben Zelltyps, ist.

8. Verfahren gemäß Anspruch 7, bei dem Apoptose dadurch festgestellt wird, daß das Rotationsspektrum der zu untersuchenden Zelle im Gegensatz zur Referenzzelle sein Maximum im Frequenzbereich von 5 bis 100, bevorzugt 6 bis 50 MHz, besonders bevorzugt 8 bis 15 MHz aufweist.

9. Verfahren gemäß Anspruch 6, bei dem Apoptose dadurch festgestellt wird, dass das Rotationsspektrum der zu untersuchenden Zelle sein Maximum in einem vergleichbaren Frequenzbereich wie die Referenzzelle aufweist, wobei die Referenzzelle ein gezielt in den Zustand der Apoptose gebrachte Zelle, bevorzugt desselben Zelltyps, ist.

10. Verfahren gemäß Anspruch 9, bei dem Apoptose dadurch festgestellt wird, daß das Rotationsspektrum der zu untersuchenden Zelle genau wie die Referenz sein Maximum im Frequenzbereich von 5 bis 100, bevorzugt 6 bis 50 MHz, besonders bevorzugt 8 bis 15 MHz aufweist.

11. Verfahren gemäß Anspruch 1, bei dem mindestens ein Dielektrophoresespektrum der mindestens einen Zelle durch Erfassung der Wandergeschwindigkeit oder der elektrophoretischen Beweglichkeit der Zelle in Abhängigkeit von der Frequenz des elektrischen Feldes gemessen wird.

12. Verfahren gemäß mindestens einem der Ansprüche 1 oder 11, bei dem das Dielektrophoresespektrum der mindestens einen Zelle bei einer externen Leitfähigkeit von insbesondere 0.3 S/m im Frequenzbereich von 0 bis 5 MHz, bevorzugt 1 bis 4 MHz, aufgezeichnet wird.

13. Verfahren gemäß Anspruch 12, bei dem Apoptose dadurch festgestellt wird, daß der Frequenzdurchtritt des Überganges von negativer zu positiver Dielektrophorese im Dielektrophoresespektrum im Bereich von 3,3 bis 3,8 MHz, bevorzugt bei 3,5 MHz, liegt.

14. Verfahren gemäß mindestens einem der Ansprüche 1, 11, 12 oder 13, bei dem Apoptose dadurch bestimmt wird, daß das Dielektrophoresespektrum der mindestens einen zu untersuchenden Zelle mit dem Dieelektrophoresespektrum einer Referenzzelle verglichen wird.

15. Verfahren gemäß Anspruch 1, bei dem mindestens ein Impedanzspektrum der mindestens einen Zelle durch Erfassung der Impedanz (Betrag und/oder Phase) der mindestens einen Zelle in Abhängigkeit von der Frequenz des Impedanzprüffeldes ein Impedanzspektrum gemessen wird.

16. Verfahren gemäß mindestens einem der Ansprüche 1 oder 15, bei dem das Impedanzspektrum der mindestens einen Zelle im Frequenzbereich von 1 Hz bis 100 kHz bevorzugt 0,5 kHz bis 10 kHz aufgezeichnet wird.

17. Verfahren gemäß mindestens einem der Ansprüche 15 oder 16, bei dem Apoptose dadurch bestimmt wird, daß das Impedanzspektrum der mindestens einen zu untersuchenden Zelle mit dem Impedanzspektrum einer Referenzzelle verglichen wird.

18. Verfahren gemäß mindestens einem der Ansprüche 6, 14 oder 17 bei dem es sich bei der Referenzzelle um eine vitale Zelle, eine gezielt in den Zustand der Apoptose gebrachte Zelle, oder eine gezielt in den Zustand der Nekrose gebrachte Zelle, jeweils bevorzugt desselben Zelltyps wie die zu untersuchende Zelle, handelt.

19. Verfahren gemäß Anspruch 18, bei dem Apoptose dadurch festgestellt wird, das das Impedanzspektrum einer apoptotischen Zelle im Gegensatz zu dem einer vitalen oder nekrotischen Zelle Änderungen im Frequenzbereich von 1 Hz bis 100 kHz bevorzugt 0.5 kHz bis 10 kHz zeigt.

20. Verfahren gemäß mindestens einem der Ansprüche 1 bis 19, bei dem die mindestens eine Zelle bei Aufnahme der Rotationsgeschwindigkeit und/oder dielektrophoretischen Beweglichkeit und/oder Impedanz bei mindestens einer Frequenz gleichzeitig suspendiert im Fokus einer optischen Falle gehalten wird.

21. Verfahren gemäß mindestens einem der Ansprüche 1 bis 20, bei dem Rotationsspektren und/oder die mindestens 2 Rotationsgeschwindigkeiten und/oder die Dielektrophoresespektren und/oder die Impedanzspektren in Abhängigkeit von der Zeit aufgenommen werden.

22. Verfahren gemäß mindestens einem der Ansprüche 1 bis 22, bei dem das Verfahren, ggf. bei der Durchführung von Chemotherapie, Radiotherapie, Immuntherapie, Operationen oder einer Kombination dieser Therapien, zur Diagnose und/oder Therapiekontrolle von Krankheiten und/oder Prozessen verwendet wird,

   - die mit einer Erhöhung der Apoptoserate verbunden sind, wie insbesondere AIDS, neurodegenerative Erkrankungen, insbesondere Alzheimer, Parkinson; durch Gifte, insbesondere Alkohol, verursachte Leberkrankheiten; Krankheiten, die eine zu geringe Hormonproduktion oder -ausschüttung hervorrufen,
   - die mit einer Erniedrigung der Apoptoserate verbunden sind, wie insbesondere maligne und benigne hyperproliferative Erkrankungen, wie Krebs, hormon-abhängige Tumore, Leukämie; Autoimmunkrankheiten, insbesondere Arthritis, Diabetis mellitus und virale Infektionen, insbesondere durch Herpesviren, Poxviren oder Adenoviren hervorgerufene Krankheiten.

23. Verfahren gemäß mindestens einem der Ansprüche 1 bis 22, bei dem eine Identifizierung von Apoptose-beeinflussenden Substanzen erfolgt.

24. Verfahren gemäß Anspruch 21, bei dem die folgenden Schritte vorgesehen sind:

   - Bereitstellen mindestens einer zu untersuchenden Zellkulturprobe,
   - Zugabe einer potentiellen Apoptose-beeinflussenden Substanz oder Mischungen aus mindestens zwei dieser Substanzen,
   - Einbringen der mindestens einen Zelle der zu untersuchenden Zellkulturprobe in ein Mikrosystem und Ermittlung des mindestens einen Messparameters, der für den Vitalitätszustand der Zelle charakteristisch ist,
   - Bestimmung der Apoptose-beeinflussenden Eigenschaften durch Vergleich des Verhaltens dieser mindestens einen Zelle im elektrischen Feld mit dem mindestens einer Zelle einer Referenzprobe.

25. Verfahren gemäß Anspruch 24, bei dem als Referenzprobe eine Probe desselben Zelltyps wie die zu untersuchende Probe, vor Zugabe der mindestens einen potentiellen Apoptose beeinflussenden Substanz, verwendet wird.

26. Verfahren gemäß mindestens einem der Ansprüche 21 bis 23, bei dem die Apoptose-beinflussenden Eigenschaften der mindestens einen Substanz dadurch festgestellt werden, dass das Rotationsspektrum der mindestens einen zu untersuchenden Zelle der Probe im Vergleich zum Spektrum der mindestens einen Zelle der Referenzprobe sein Maximum in einem niedrigen Frequenzbereich aufweist.

27. Verfahren gemäß Anspruch 26, bei dem die Apoptose-beeinflussenden Eigenschaften der mindestens einen Substanz dadurch festgestellt werden, daß das Rotationsspektrum der mindestens einen zu untersuchenden Zelle der Probe im Gegensatz zum Spektrum der mindestens einen Zelle der Referenzprobe eine Änderung insbesondere im Frequenzbereich von 5 bis 100, bevorzugt 6 bis 50 MHz, besonders bevorzugt 8 bis 15 MHz und/oder im Frequenzbereich von 1-4 MHz, bevorzugt 2 bis 3 MHz, besonders bevorzugt 2,3-2,6 MHz aufweist.

28. Verfahren gemäß mindestens einem der Ansprüche 23 bis 27, bei dem eine dielektrische Separation der Zellen eines bestimmten Volumens der Probe und/oder der eines bestimmten Volumens der Referenzprobe durchgeführt wird.

29. Verfahren gemäß Anspruch 28, bei dem nach Durchführung der dielektrischen Separation, die Anzahl der apoptotischen Zellen der zu untersuchenden Probe im Verhältnis zu der Anzahl der apoptotischen Zellen der Referenzprobe ermittelt wird.

30. Verfahren gemäß mindestens einem der Ansprüche 23 bis 29, bei dem Apoptose-erhöhende Substanzen durch eine Erhöhung der Anzahl apoptotischer Zellen im Verhältnis zur Referenzprobe charakterisiert sind.

31. Verfahren gemäß mindestens einem der Ansprüche 21 bis 30, bei dem Substanzen, die die Apoptose nicht beeinflussen, durch einen zur Referenzprobe vergleichbaren Anteil apoptotischer Zellen charakterisiert sind.

32. Verfahren gemäß mindestens einem der Ansprüche 21 bis 31, bei dem Apoptose-hemmende Substanzen durch eine niedrigere Anzahl apoptotischer Zellen im Verhältnis zur Referenzprobe charakterisiert sind.

33. Verfahren gemäß mindestens einem der Ansprüche 21 bis 32, bei dem die Zellkulturprobe vor oder während der Zugabe einer potentiellen Apoptose-hemmenden Substanz mit einer Apoptose-erhöhenden Substanz versetzt wird.

34. Verfahren gemäß Anspruch 33, bei dem als Referenzprobe die mit einer Apoptose-erhöhenden Substanz versetzte Zellkulturprobe und oder eine Zellkulturprobe, die den gleichen Anteil Apoptose-erhöhende Substanz erhält, eingesetzt wird.

35. Verfahren gemäß Anspruch 34, bei dem eine Apoptose-hemmende Substanz dadurch charakterisiert ist, daß sie im Verhältnis zur Referenzprobe einen geringeren Anteil apoptotischer Zellen aufweist.

**A**

**B**

Figur 1

Figur 2

Figur 3

Figur 4

Figur 6

Figur 5

Europäisches **EUROPÄISCHER TEILRECHERCHENBERICHT**     Nummer der Anmeldung
Patentamt     der nach Regel 45 des Europäischen Patentübereinkommens für das weitere Verfahren als     EP 00 12 4662
europäischer Recherchenbericht gilt

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X,D | REICHLE ET AL: BIOCHIMICA ET BIOPHYSICA ACTA, Bd. 1459, Nr. 1, 20. Juli 2000 (2000-07-20), Seiten 218-29, XP001000323 * Zusammenfassung * * Seite 224, linke Spalte, Absatz 2 - Seite 226, linke Spalte, Zeile 2; Abbildung 6 * —/— | 1-10, 20-35 | C12M1/34 |

**RECHERCHIERTE SACHGEBIETE (Int.Cl.7)**

C12M

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:
Siehe Ergänzungsblatt C

| Recherchenort | Abschlußdatum der Recherche | Prüfer |
|---|---|---|
| MÜNCHEN | 31. Mai 2001 | Luis Alves, D |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

**Europäisches
Patentamt**

**UNVOLLSTÄNDIGE RECHERCHE
ERGÄNZUNGSBLATT C**

Nummer der Anmeldung

EP 00 12 4662

Obwohl der Anspruch 22 sich auf ein Verfahren zur Behandlung des
menschlichen/tierischen Körpers bezieht (Artikel 52(4) EPÜ), wurde die
Recherche durchgeführt und gründete sich auf die angeführten Wirkungen
der Verbindung/Zusammensetzung. Das gleiche gilt für Ansrpuch 23, der auf
Ansprüch 22 rückbezogen ist.

Europäisches
Patentamt

**EUROPÄISCHER
TEILRECHERCHENBERICHT**

Nummer der Anmeldung

EP 00 12 4662

| | **EINSCHLÄGIGE DOKUMENTE** | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| X | BONINCONTRO A ET AL: "Apoptosis dependent decrease of the intramembrane ion traffic in cultured mouse fibroblasts shown by conductivity dispersion" BIOSCIENCE REPORTS, (DEC 1997) VOL. 17, NO. 6, PP. 547-556. PUBLISHER: PLENUM PUBL CORP, 233 SPRING ST, NEW YORK, NY 10013. ISSN: 0144-8463., XP001000475 UNIV ROMA LA SAPIENZA, DIPARTIMENTO GENET & BIOL MOL, PIAZZALE ALDO MORO 5, I-00185 ROME, ITALY (Reprint);UNIV ROMA LA SAPIENZA, DIPARTIMENTO GENET & BIOL MOL, I-00185 ROME, ITALY; INFM, DIPARTIMENTO FIS, I-00185 ROME, ITALY * Seite 548, letzter Absatz – Seite 549, letzter Absatz; Abbildungen 2,4 * * Seite 553, Absatz 2 * | 1,3, 15-25 | |
| | --- | | |
| X | HOLZEL R.: BIOCHIMICA ET BIOPHYSICA ACTA, Bd. 1425, Nr. 2, 23. Oktober 1998 (1998-10-23), Seiten 311-318, XP001000317 * Zusammenfassung * * Seite 311, linke Spalte, Zeile 1 – rechte Spalte, Zeile 2 * * Seite 312, linke Spalte, Absatz 2 * * Seite 313, rechte Spalte, Absatz 2 – Seite 317, rechte Spalte, letzter Absatz; Abbildungen 2,3 * | 1-10, 20-35 | RECHERCHIERTE SACHGEBIETE (Int.Cl.7) |
| | --- | | |
| X | WANG X J ET AL: BLOOD, Bd. 92, Nr. 10 suppl. 1, 15. November 1998 (1998-11-15), Seite 167b XP001000341 * Zusammenfassung * | 1,11-25 | |
| | --- | | |
| | -/-- | | |

**Europäisches Patentamt**

**EUROPÄISCHER TEILRECHERCHENBERICHT**

Nummer der Anmeldung

EP 00 12 4662

| | EINSCHLÄGIGE DOKUMENTE | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| A | WO 99 09208 A (COULTER INT CORP) 25. Februar 1999 (1999-02-25) * Ansprüche * | 1-35 | |
| A | US 6 004 767 A (CROUCH SHARON PATRICIA MARY ET AL) 21. Dezember 1999 (1999-12-21) * Ansprüche * | 1-35 | |

**RECHERCHIERTE SACHGEBIETE (Int.Cl.7)**

EPO FORM 1503 03.82 (P04C12)

**ANHANG ZUM EUROPÄISCHEN RECHERCHENBERICHT
ÜBER DIE EUROPÄISCHE PATENTANMELDUNG NR.**

EP 00 12 4662

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-05-2001

| Im Recherchenbericht angeführtes Patentdokument | Datum der Veröffentlichung | Mitglied(er) der Patentfamilie | Datum der Veröffentlichung |
|---|---|---|---|
| WO 9909208 A | 25-02-1999 | US 5976822 A | 02-11-1999 |
| US 6004767 A | 21-12-1999 | EP 0948641 A | 13-10-1999 |
| | | WO 9828437 A | 02-07-1998 |
| | | GB 2323167 A,B | 16-09-1998 |

Für nähere Einzelheiten zu diesem Anhang : siehe Amtsblatt des Europäischen Patentamts, Nr. 12/82